# EUROPÄISCHE PATENTSCHRIFT

(11) **EP 2 773 951 B1**
(45) Veröffentlichungstag und Bekanntmachung des Hinweises auf die Patenterteilung: **10.02.2016**
(21) Anmeldenummer: 12769036.0
(22) Anmeldetag: 30.08.2012
(51) Int. Cl.: G01N 33/00

(54) **MESSGERÄT UND VERFAHREN ZUM ERFASSEN DES KOHLENWASSERSTOFFANTEILS IN GASEN**
MEASUREMENT DEVICE AND METHOD FOR DETECTING THE HYDROCARBON CONTENT IN GASES
APPAREIL DE MESURE ET PROCÉDÉ PERMETTANT DE DÉTECTER LA PROPORTION D'HYDROCARBURES DANS DES GAZ

(30) Priorität: 02.11.2011 DE 102011055001
(43) Veröffentlichungstag der Anmeldung: 10.09.2014
(73) Patentinhaber: BEKO TECHNOLOGIES GMBH, 41468 Neuss (DE)
(72) Erfinder: FRIEDRICH, Martin, 79843 Löffingen (DE)
(74) Vertreter: Patentanwälte Bauer Vorberg Kayser
(86) Internationale Anmeldenummer: PCT/EP2012/066812
(87) Internationale Veröffentlichungsnummer: WO 2013/064280

(56) Entgegenhaltungen:
- WO-A1-2010/094750
- WO-A2-2010/075858
- DE-A1- 2 626 905
- US-A- 3 558 283
- US-A- 4 388 411

## Beschreibung

Die vorliegende Erfindung betrifft ein Messgerät und ein Verfahren zur Erfassung des Kohlenwasserstoffanteils in Gasen.

Derartige Messgeräte sind mit verschiedenen Sensortechnologien bekannt und dienen der Erfassung des Gehaltes von Öl, Kohlenwasserstoffen und oxidierbaren Gasen beispielsweise in Luft oder Druckluft. Häufig werden beispielsweise elektrisch beheizbare Halbleiter-Oxidmaterialien verwendet, die im beheizten Zustand ihren elektrischen Widerstand in Abhängigkeit von der Menge der in der Luft enthaltenen Kohlenwasserstoffe verändern.

Eine weitere Methode ist die Erfassung von Kohlenwasserstoffen mittels Pellistoren. Dazu wird der zu messende Gasstrom über einen Körper aus beheiztem Katalysatormaterial geleitet, in dessen Inneren sich eine beheizte Platinwendel befindet. Die Kohlenwasserstoffkonzentration ist über die Änderung des elektrischen Widerstandes der beheizten und einer zweiten Platinwendel erfässbar, die sich durch die Verbrennungswärme des Kohlenwasserstoffanteils am Katalysator einstellt.

Ebenfalls bekannt ist die Verwendung von Flammenionisationsdetektoren. Bei derartigen Vorrichtungen werden die Kohlenwasserstoffen in einem Gasstrom verbrannt und der Ionisationsstrom zwischen zwei Elektroden in der Flamme gemessen.

Eine weitere Methode ist die Erfassung der Kohlenwasserstoffkonzentration mittels Photoionisation. Dabei werden die Kohlenwasserstoffe mit ultraviolettem Licht bestrahlt. Die Energiemenge des Lichts muss dabei so hoch sein, dass Elektronen aus dem Kohlenwasserstoffmolekül herausgetrieben werden. Deren Anzahl lässt sich elektronisch messen.

Die oben genannten Verfahren eignen sich insbesondere für die Detektion höherer Konzentrationen in oxidierbaren Gasen, die Detektion geringerer Konzentrationen im unteren µg/m³-Bereich bzw. im ppb-Bereich ist aber nicht zuverlässig möglich.

Die mittels Photoionisationsdetektoren generierten Messwerte lassen nur indirekt auf die gemessene Stoffmenge schließen, da die Messwerte auch vom molekularen Aufbau der Verbindung abhängig sind und selbst bei gleichen Summenformeln recht stark variieren. Sofern die zu messende Verbindung aber konstant, bekannt und möglichst auch einheitlich ist, lässt sich die Konzentration des Kohlenwasserstoffanteils relativ zuverlässig messen. Allerdings sinkt die Messgenauigkeit mit abnehmender Konzentration an Kohlenwasserstoffen. Insbesondere steigt dabei der Einfluss des Feuchtegehalts der Luft. Mit abnehmendem Kohlenwasserstoffanteil wird der Einfluss der Luftfeuchte zunehmend größer, Messungen von Kohlenwasserstoffanteilen im unteren mg/m³-Bereich und insbesondere im µg/-m³-Bereich sind nicht ausreichend genau durchzuführen.

Für die unterschiedlichen Anwendungen von Druckluft werden unterschiedliche Grenzwerte für den Ölanteil gefordert. Ölanteile bestehen aus tröpfchenförmigen Ölerosolen und aus Öldämpfen. Ölerosole und Öldämpfe können durch verschiedene Verfahren aus dem Druckluftstrom teilweise oder weitgehend eliminiert werden.

Die WO 2010/094750 beschreibt ein Messgerät und ein Verfahren zum Erfassen des Kohlenwasserstoffanteils in Gasen. Ein Ursprungsgasstrom wird in einen ersten Gasstrom und einen zweiten Gasstrom unterteilt, die beide von einem geeigneten Messgerät untersucht werden. Wesentlich ist, dass der erste Gasstrom unverändert und der zweite Gasstrom aufbereitet an das Messgerät geleitet werden. Der Kohlenwasserstoffanteil wird dabei vorzugsweise über eine Signaldifferenz zwischen dem ersten Gasstrom und dem zweiten Gasstrom ermittelt. Die beschriebene Technologie ermöglicht zwar bereits eine sehr zuverlässige Messung auch geringster Konzentrationen, allerdings ist die Zuverlässigkeit der Technologie noch nicht ausreichend. Störungen des Messgerätes oder Fehler in den Zuleitungen, die das Messergebnis beeinflussen können, werden, wenn überhaupt, nur mit hohem Zeitverzug erkannt. Störungen oder Defekte im System können darüber hinaus nur mit erheblichem Aufwand identifiziert und behoben werden, was in der Regel ein abschalten der gesamten Anlage notwendig macht.

Das Dokument US 4 388 411 beschreibt ein weiteres artverwandtes Messgerät.

Die Aufgabe der Erfindung besteht darin, ein Messgerät zur Erfassung des Gehalts von Öl, Kohlenwasserstoffen und oxidierbaren Gasen in Gasen zu schaffen, dass zum Einen auch geringste Konzentrationen zuverlässig misst und zum Anderen die Nachteile des Standes der Technik nicht aufweist. Das Messgerät soll insbesondere eine geringe Fehleranfälligkeit aufweisen bzw. ermöglichen, Messfehler und/oder Störungen einfach und effektiv zu lokalisieren. Weiterhin ist es Aufgabe der Erfindung ein gegenüber dem Stand der Technik verbessertes Verfahren zur Erfassung des Gehaltes von Öl, Kohlenwasserstoffanteilen und oxidierbaren Gasen in Gasen bereit zu stellen.

Die Aufgabe wird gelöst, durch Messgerät zur Erfassung von Kohlenwasserstoffanteilen in Gasen, aufweisend
- Vorrichtungen zum Aufteilen eines zu messenden Ursprungsgasstroms in einen ersten Messgasstrom und einen zweiten Messgasstrom,
- eine erste Referenzgaseinheit zur Erzeugung eines ersten Referenzgasstroms aus einem Teilstrom des ersten Messgasstroms,
- eine zweite Referenzgaseinheit zur Erzeugung eines zweiten Referenzgasstroms aus einem Teilstrom des zweiten Messgasstroms,
- einen ersten Sensor zur Bestimmung des Anteils an Kohlenwasserstoff im ersten Messgasstrom und zur Erzeugung eines entsprechenden ersten Messergebnisses,
- einen zweiten Sensor zur Bestimmung des Anteils an Kohlenwasserstoff im zweiten Messgasstrom und zur Erzeugung eines entsprechenden zweiten Messergebnisses,
- einem Sensor zum Messen des Volumenstroms
- eine Auswerteinheit zur Auswertung der Messergebnisse der beiden Sensoren,
   wobei
- dem ersten Sensor im Wechsel der erste Messgasstrom oder der von der ersten Referenzgaseinheit aufbereitete erste Referenzgasstrom zugeführt wird,
- dem zweiten Sensor im Wechsel der zweite Messgasstrom oder der von der zweiten Referenzgaseinheit aufbereitete zweite Referenzgasstrom zugeführt wird.

Weiterhin wird die Aufgabe durch ein Verfahren zum Erfassen des Kohlenwasserstoffanteils in einem Gasstrom gelöst, das gekennzeichnet ist durch die Verfahrensschritte:
- Aufteilen eines zu messenden Ursprungsgasstroms in einen ersten Messgasstrom und einen zweiten Messgasstrom,
- Erzeugen eines ersten Referenzgasstroms aus einem Teilstrom des ersten Messgasstroms,
- Erzeugen eines zweiten Referenzgasstroms aus einem Teilstrom des zweiten Messgasstroms,
- Wechselseitiges Zuführen des ersten Messgasstroms oder des von der ersten Referenzgaseinheit aufbereiteten ersten Referenzgasstroms zum ersten Sensor,
- Wechselseitiges Zuführen des zweiten Messgasstrom oder des von der zweiten Referenzgaseinheit aufbereiteten zweiten Referenzgasstroms zum zweiten Sensor,
- Bestimmen des Anteils an Kohlenwasserstoff im ersten Messgasstrom und Erzeugen eines entsprechenden ersten Messergebnisses,
- Bestimmen des Anteils an Kohlenwasserstoff im zweiten Messgässtrom und zur Erzeugen eines entsprechenden zweiten Messergebnisses,
- Auswerten der Messergebnisse der beiden Sensoren.

Erfindungsgemäß wird der zu messende Ursprungsgasstrom in einen ersten Messgasstrom und einen zweiten Messgasstrom aufgeteilt. Das Aufteilen des Ursprungsgasstroms kann dabei durch ein tatsächliches physikalisches Aufteilen beispielsweise mittels eines Separators erfolgen, alternativ kann der Ursprungsgasstrom beispielsweise mit Hilfe von Ventilen wechselweise dem ersten Sensor der dem zweiten Sensor bzw. den jeweiligen Referenzgaseinheiten zugeführt werden. Als Referenzgaseinheiten sind beispielsweise Katalysatoren, insbesondere Oxidationskatalysatoren einsetzbar.

Erfindungsgemäß weist das Messgerät im Gegensatz zu den bekannten Messgeräten nicht nur einen, sondern zwei Sensoren zur Messung von Kohlenwasserstoffanteilen auf. Die beiden Sensoren werden wechselweise mit Referenzgas oder Messgas beaufschlagt. Beide Sensoren ermitteln ihr Messergebnis auf diese Weise jeweils als Signaldifferenz zwischen dem Messgas und dem erzeugten Referenzgas, also dem oxidierten Messgas. Die Messergebnisse können neben dem Anteil an Kohlenwasserstoffen auch andere Messwerte enthalten.

Die Erfindung basiert auf der Idee, zumindest zwei Messgaswege vorzusehen und entsprechend zumindest zwei Messergebnisse zu erzeugen. Es wird explizit darauf hingewiesen, dass die Erfindung nicht auf die Verwendung zweier Sensoren festgelegt sein soll, sondern dass darüber hinaus weitere Messgaswege und Sensoren möglich sind. Die Erfindung wird im Folgenden nur beispielhaft für die Verwendung zweier Sensoren erläutert.

Zu Beginn der Messung, beispielsweise beim Start des Messgeräts, können in einer Anlaufphase zunächst in beiden Messgaswegen die Sensoren mit Referenzgas beaufschlagt werden. Nach einer gewissen Zeit schaltet der erste Gasweg auf Messgas um, während dem Sensor im zweiten Gasweg weiterhin Referenzgas zugeführt wird. Anschließend wird gewechselt, so dass dem ersten Sensor im ersten Gasweg Referenzgas und dem zweiten Sensor im zweiten Gasweg Messgas zugeführt wird. Dieser Wechsel wird beliebig oft, vorzugsweise in relativ kurzen Zeitintervallen, wiederholt. Eine Zeitdauer von etwa 5 bis30, vorzugsweise 20 s pro Zyklus hat sich als ausreichend und sinnvoll erwiesen.

Als besonders vorteilhaft hat es sich erwiesen, das Intervall in Abhängigkeit von der Gaskonzentration zu verändern. Bei geringer Konzentration im ppb Bereich kann das Intervall vorzugsweise 1, bis 50 Sekunden, insbesondere ca. 30 Sekunden betragen, bei großen Konzentrationen (z.B. 2 ppm) dagegen etwa 80 bis 150, vorzugsweise etwa 120 Minuten.

Das Messgerät weist eine Auswerteinheit zur Auswertung der Messergebnisse auf. In einer ersten Ausführungsvariante können die beiden Sensoren mit einer einzigen Auswerteeinheit verbunden sein, denkbar ist aber auch, dass jedem Sensor eine eigene Auswerteeinheit zugeordnet ist. Die Auswerteeinheit weist einen Prozessor auf, der die notwendigen Berechnungen durchführt. Es können die einzelnen Gasströme oder die Summe aller Gasströme durch den Sensor bzw. die Sensoren gemessen oder von der Auswerteeinheit ausgewertet werden.

Weiterhin ist eine Anzeigeeinheit vorgesehen, die die Messergebnisse der Sensoren oder von der Auswerteeinheit berechnete Werte und/oder weitere Informationen anzeigt. Die Anzeigeeinheit kann vorteilhafterweise auch als Eingabeeinheit in Form eines Touchscreens ausgeführt sein.

Erfindungsgemäß ist es nun möglich, dass die beiden Sensoren ihre Messergebnisse nach einer bestimmten Abfolge von Schritten zur Auswertung und/oder zur Anzeige bringen. Die Anzeigewerte, die von der Anzeigeeinheit dargestellt werden, liegen also kontinuierlich vor. Zunächst wird das Messergebnis des ersten Sensors angezeigt, anschließend das Messergebnis des zweiten Sensors. Die Messzyklen der Sensoren sind derart zeitversetzt, dass ein kontinuierliches Messsignal bereitsteht.

Es hat sich als besonders vorteilhaft und für den Benutzer als angenehm erwiesen, wenn das Umschalten der Anzeigewerte zeitversetzt zum Umschalten der Gaswege und zur Beaufschlagung der Sensoren erfolgt. Auf der Anzeigeeinheit ist somit noch das Messergebnis des ersten Sensors erkennbar, während der zweite Sensor bereits mit Messgas beaufschlagt und die Messung begonnen wurde. Erst wenn ein zuverlässiges Messergebnis des zweiten Sensors erreicht ist, wird die Anzeigeeinheit von der Auswerteeinheit angesprochen und entsprechend umgeschaltet. Somit wird stets ein zuverlässig gemessenes Messergebnis angezeigt. Dies ist insbesondere auch deshalb von Vorteil, weil die Sensoren üblicherweise eine gewisse Einschwingphase benötigen, bevor sie ein stabiles Messergebnisse liefern. Durch die zeitversetzte Anzeige ist die Einschwingphase, in der das Messergebnis verhältnismäßig stark schwankt, für den Benutzer nicht sichtbar.

Die Verwendung zweier Sensoren ermöglicht es aber auch, dass diese stets gegeneinander abgeglichen werden können. Wird eine Abweichung erkannt, kann diese verschiedene Ursachen haben. Beispielsweise kann sich die Zusammensetzung des Messgases über die Zeit tatsächlich geändert haben. Möglich ist aber auch, dass einer der Sensoren fehlerhafte Messergebnisse liefert. Schließlich können die Gaswege Verunreinigungen oder Undichtigkeiten aufweisen.

Erfindungsgemäß kann die Auswerteeinheit entsprechende Rechenoperationen und Routinen durchführen, um die Ursache der Abweichung zu ermitteln. Zeigt sich beispielsweise, dass nach jedem Wechsel der Sensoren eine Abweichung auftritt, kann eine Änderung der Zusammensetzung des Messgases nahezu ausgeschlossen werden. In einer besonders vorteilhaften Ausführungsvariante sind beide Sensoren an beide Gaswege angeschlossen, so dass in einem nächsten Abgleich durch einen Tausch der Gaswege ermittelt werden kann, ob die Abweichung durch die Gaswege oder durch die Sensoren begründet ist.

Vorteilhafterweise führt die Auswerteeinheit ein entsprechendes Fehleranalyseprogramm selbsttätig durch und steuert einzelne Bauteile des Messgeräts, wie beispielsweise Ventile und/oder Drosseln, um eine Überprüfung sämtlicher Elemente der Gaswege und Sensoren durchzuführen. Dadurch, dass bei Bedarf jeder Gasstrom einzeln geschaltet werden kann und am Austritt der Volumenstrom gemessen wird, sind sehr detaillierte Fehleranalysen möglich. Es ist auch möglich, dem Benutzer die Ursache eines Fehlers über die Anzeigeeinheit darzustellen.

Die Auswerteeinheit ist auch in der Lage, einen der beiden fehlerhaften Sensoren oder Gaswege selbstständig abzuschalten, so dass das Messgerät weiterhin einsatzbereit bleibt. Vorteilhafterweise gibt die Auswerteeinheit eine entsprechende Information über die Anzeigeeinheit aus, so dass das Messgerät vor Ausfall des zweiten Gasweges oder Sensors und dem damit verbundenen Gesamtausfall repariert werden kann. Ein wesentlicher Vorteil ergibt sich auch daraus, dass somit die entsprechende Reparatur während einer späteren ohnehin anstehenden Betriebspause durchführbar ist.

Letztendlich ist das Messgerät mit einem entsprechenden Fehleranalyseprogramm in der Lage, sämtliche Bauteile im Gasweg selbstständig zu überprüfen. Auch ein Spülen der Sensoren mit Referenzgas kann in regelmäßigen Zyklen oder aufgrund eines abweichenden oder auffälligen Messergebnisses vom Messgerät selbstständig eingeleitet werden.

Das erfindungsgemäße Messgerät kann weiterhin derart ausgeführt sein, dass beide Sensoren jeweils mit einem Kalibriergas, beispielsweise aus einer Vorratsflasche, beaufschlagt werden können. Neben dem unveränderten Messgas und dem Referenzgas wird also weiterhin regelmäßig ein Kalibriergas beispielsweise zur Neufestlegung der vom Sensor ausgehenden Signalstärke verwendet. Das Kalibriergas (z.B. Isobuten) weist einen definierten Kohlenwasserstoffanteil auf, allerdings keine oder nur eine ausgesprochen geringe Feuchtigkeit. Erfindungsgemäß ist es somit möglich, die Veränderung der Signalstärke und Messempfindlichkeit durch Alterung und Verschmutzung des Messgeräts zuverlässig auszugleichen. Die Kalibriermessung kann in regelmäßigen Abständen automatisch erfolgen, sie kann aber auch jederzeit vom Anwender eingeleitet werden. Insbesondere kann sie im Rahmen der Fehleranalyse vom Fehleranalyseprogramm genutzt werden. Die im Rahmen der Kalibriermessung ermittelten Daten werden gespeichert und können jederzeit abgerufen, und von der Auswerteinheit genutzt werden.

Es ist darüber hinaus durch einen Abgleich bzw. Vergleich der ermittelten Messergebnisse miteinander oder mit gespeicherten Messergebnissen (beispielsweise aus einer Referenzdatenbank) möglich, eine Kalibrierung der Sensoren bei einer Beaufschlagung mit Messgas, Referenzgas oder auch Kalibriergas vorzunehmen. Dabei beschreibt der Begriff Messgas zu messendes Gas mit unbekannter ppm Konzentration, der Begriff Referenzgas bekanntes Gas mit 0 ppm Konzentration der Begriff Kalibriergas bekanntes Gas mit bestimmter ppm Konzentration.

Vorteilhafterweise können vor der Inbetriebnahme des Messgeräts die beiden Sensoren durch entsprechende Zufuhr von Kalibriergas auch derart eingestellt werden, dass sie trotz bautechnischer Abweichung oder Alterung das gleiche Messergebnis liefern.

Erfindungsgemäß kann jeder Messzyklus eine Kalibrierphase aufweisen, in der die aktuell vom einzelnen Sensor gemessenen Messwerte überprüft und verglichen werden.

Ein Messzyklus beginnt beispielsweise mit der Zuführung von Referenzgas. Nach einer Einschwingphase wird ein konstantes Messergebnis geliefert und der entsprechende Sensor mit dem Referenzgas gespült. In der anschließenden Kalibrierphase werden die zuvor gemessenen Messergebnisse beispielsweise mit bei der Herstellung für diesen Sensor erfassten Messergebnisse verglichen und der Sensor wird gegebenenfalls kalibriert. Im Rahmen der Kalibrierphase sind auch weitere sinnvolle Abgleiche möglich, zum Beispiel mit dem Messergebnis des anderen Sensors bei entsprechender Beaufschlagung mit Referenzgas. Es folgt ein Umschalten auf Messgas und erneut einer Einschwingphase über einen kurzen Zeitraum. Im anschließenden stabilen Betriebszustand, in dem ein zuverlässiges Messergebnis geliefert werden kann, erfolgt der Vergleich des Messergebnisses mit dem unmittelbar zuvor gemessenen Messergebnis und/oder mit einem gespeicherten Messergebnis des anderen Sensors und/oder einem weiteren geeigneten Referenzwert. Der Messzyklus ist beendet und es erfolgt mit Umschalten auf Referenzgas der Beginn des nächsten Messzyklus. Der gleiche Messzyklus läuft zeitversetzt im anderen Gasweg mit dem anderen Sensor ab.

Die Auswerteeinheit kann erfindungsgemäß auch stets einen Mittelwert der Messergebnisse der beiden Sensoren errechnen und diesen über die. Anzeigeeinheit darstellen. Erst bei Überschreiten einer voreingestellten Abweichung reagiert das Messgerät. Beispielsweise startet dann das oben bereits beschriebene Fehleranalyseprogramm und/oder es werden lediglich beide Messergebnisse abwechselnd oder gleichzeitig, vorzugsweise verbunden mit einem Alarm, angezeigt. In einer besonders einfachen Ausführungsvariante wird weiterhin der Mittelwert angezeigt und ein Alarm ausgelöst.

Das Messgerät kann dann besonders kostengünstig hergestellt werden, wenn technische Einheiten in Form von Baublöcken zusammengefasst werden. Dies kann beispielsweise Ventile, Drosseln, Katalysatoren und Sensoren betreffen. Die entsprechenden Elemente und Bauteile bestehen beispielsweise aus Metall.

In einer besonders vorteilhaften Ausführungsvariante beruht die Erfassung der Kohlenwasserstoffanteile auf dem Prinzip der Photoionisation und weist eine entsprechende Sensoreinheit mit Probenahmesonde auf. Die Sensoreinheit ist dabei über ein Signalkabel oder kabellos mit der Auswerteeinheit verbunden. Die Probenahmesonde kann vorzugsweise von oben zentrisch in eine Steigleitung montiert sein, so dass sie mittig aus dem zu überwachenden Gasstrom Gas entnehmen kann. Die Sensoreinheit weist definierte Fließwiderstände auf, die für einen konstanten Druck und einen konstanten Volumenstrom der einzelnen Messgase sorgen und beispielsweise durch eine Drossel mit definierter Bohrung, bzw. aus einem Sintermetall gebildet sind. Diese sind besonders wartungsarm und einfach zu reinigen. Weiterhin ist eine Alarmfunktion vorgesehen, die dem Benutzer bei zu niedrigem oder zu hohem Druck der Gasströme visuell oder akustisch informiert.

Die Durchflussmenge der verschiedenen Gasströme kann mit entsprechenden Drosseln, Ventilen oder Durchflussreduktoren beeinflusst werden. Diese sind vorzugsweise austauschbar und in einer besonders vorteilhaften Ausführungsvariante regelbar, um damit zum Einen die Durchflussmenge zu den Sensoren einstellen und zum Anderen die gewünschten Mischverhältnisse der zu mischenden Gasströme zuverlässig gewährleisten zu können.

Dadurch, dass die Ventile im Gasweg einzeln schaltbar sind, ist auch möglich, Referenzluft und Messluft gleichzeitig auf den Sensor zu schalten und das Messgas zu verdünnen. Damit kann der Messbereich nach oben erweitert werden, wenn die Messluft extrem stark verschmutzt ist.

Als Referenzgaserzeuger können übliche Oxidationskatalysatoren eingesetzt werden, denkbar sind aber auch andere Vorrichtungen oder Verfahren zur Bereitstellung von Gasen mit den gewünschten Eigenschaften. Als Oxidationskatalysator dient beispielsweise platinierte Quarzwolle, die problemlos in einen dafür vorgesehenen Behälter einführbar ist. In einer besonders vorteilhaften Variante ist der Referenzgaserzeuger in das Messgerät integriert, wodurch vor Ort lediglich die verschiedenen Fluid bzw. Gaszuführungen angeschlossen werden müssen. Das Messgerät weist somit sämtliche Anschlüsse für entsprechende Gasleitungen und auch den elektrischen Anschluss auf, sodass es vor Ort flexibel an beliebigen Orten installierbar ist. Insbesondere die erfindungsgemäße Aufteilung des Messgeräts in die Sensoreinheit mit Sensoren, beispielsweise der Probenahmesonde im Falle des Prinzips der Photoionisation und die Auswerteeinheit mit Bedienoberfläche (Display) erweitert die Möglichkeiten einer räumlich flexiblen Aufstellung vor Ort zusätzlich. Die Auswerteeinheit mit Bedienoberfläche baut klein und kann nahezu überall, vorteilhafterweise an einer gut zugänglichen Position installiert werden, während die etwas größere Sensoreinheit räumlich getrennt von der Auswerteeinheit an der Messgasentnahmestelle angeordnet sein kann.

Das erfindungsgemäße Messgerät kann vorzugsweise mit einem ölfrei erdichtenden Kompressor zur Herstellung von Druckluft oder Druckgas verwendet werden, denkbar ist aber auch die Verwendung mit einem ölgeschmierten Kompressor, wenn diesem ein entsprechender Katalysator nachgeschaltet ist. Für Wartungsarbeiten ist vorzugsweise ein Bypass vorgesehen. Grundsätzlich ist das Messgerät aber auch für weitere Verwendungsbereiche geeignet, beispielsweise für Druckgasflaschen.

Die Erfindung wird im Folgenden mit Bezug auf die beiliegenden Figuren näher erläutert. Dabei zeigen die Figuren lediglich eine vorteilhafte Ausführungsvariante in stark vereinfachter Prinzipdarstellung, die Erfindung soll keinesfalls auf diese beschränkt sein. Es zeigen:
- Fig. 1:: eine vereinfachte Funktionsdarstellung des erfindungsgemäßen Messgeräts,
- Fig. 2:: ein Gaswegschaltschema des Messgeräts,
- Fig. 3:: ein Messzyklusschema des Messgeräts.

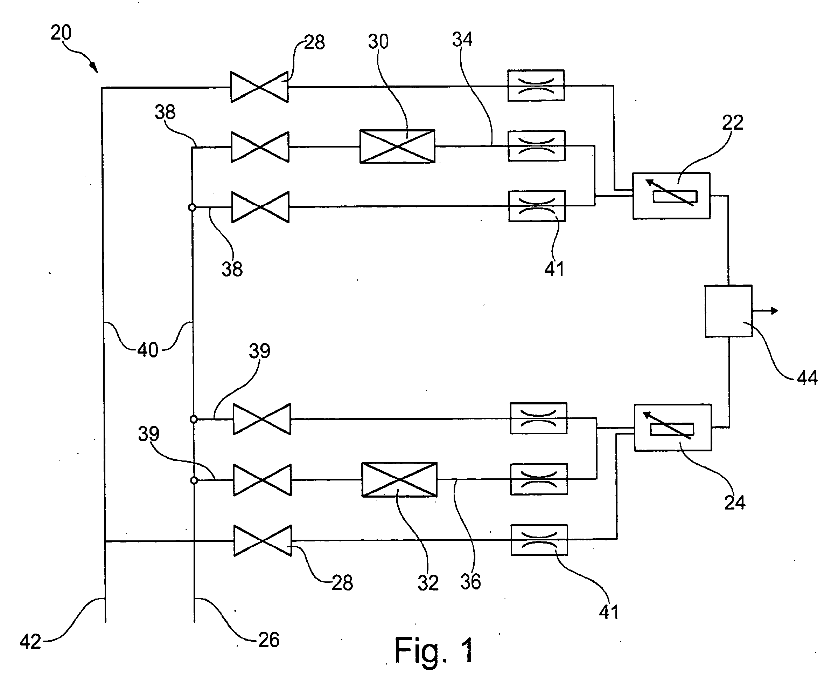

Figur 1 zeigt in einer Prinzipdarstellung die wesentlichen Elemente eines erfindungsgemäßen Messgeräts 20. Dieses weist zwei Sensoren, einen ersten Sensor 22 und einen zweiten Sensor 24, vorzugsweise Photoionisationsensoren oder Metalloxidsensoren, auf.

Ein Ursprungsgasstrom 26 wird über Gasleitungen und mit Hilfe von Ventilen 28 in einen ersten Messgasstrom 38 und einen zweiten Messgasstrom 39 aufgeteilt. Im gezeigten Ausführungsbeispiel erfolgt die Aufteilung des Ursprungsgastroms 26 über die Zeit, eine Aufteilung in zwei getrennte Volumenströme ist aber ebenfalls möglich.

Der erste Messgasstrom 38 wird alternierend teilweise dem Sensor 22 oder einer ersten Referenzgaseinheit 30 und der zweite Messgasstrom 39 alternierend teilweise dem zweiten Sensor 24 oder einer zweiten Referenzgaseinheit 32 zugeführt. Die Referenzgaseinheiten 30,32 erzeugen aus den Messgasströmen 38, 39 jeweils einen ersten Referenzgasstrom 34 bzw. einen zweiten Referenzgasstrom 36, die dann dem entsprechenden Sensor 22, 24 zugeleitet werden. Die Referenzgaseinheiten 30, 32 sind vorzugsweise als Katalysatoren, insbesondere als Oxidationskatalysatoren ausgebildet.

Dem ersten Sensor 22 wird somit im Wechsel der erste Messgasstrom 38 (entspricht dem Ursprungsgasstrom 26) oder der von der ersten Referenzgaseinheit 30 aufbereitete erste Referenzgasstrom 34 zugeführt. Entsprechend wird dem zweiten Sensor 24 im Wechsel der zweite Messgasstrom 39 (entspricht ebenfalls dem Ursprungsgasstrom 26) oder der von der zweiten Referenzgaseinheit 32 aufbereitete zweite Referenzgasstrom 36 zugeführt.

Die Gasströme 26, 34, 36, 38, 39 werden durch geeignete Gasleitungen 40 geleitet, die die entsprechenden Gaswege ausbilden. Im Verlauf der Gasleitungen 40 sind Drosseln 41 vorgesehen, die die Gasströme 26, 34, 36, 38, 39 auf ein für die Sensoren 22, 24 oder die Referenzgaseinheiten 30,32 geeignetes Druckniveau von beispielsweise 2 bar drosseln (nicht dargestellt, in Fig. 1 ist die Drossel nur symbolisch dargestellt). Eine entsprechende Drossel 41 kann auch am Anfang der Gasleitung 40 vorgesehen sein, da der Ursprungsgasstrom 26 oftmals mit sehr hohem Druck in das Messgerät 20 eingeleitet wird. Diese erste Drossel 41 senkt das Druckniveau auf beispielsweise 7 bar ab.

Neben dem Ursprungsgasstrom 26 wird über eine weitere Gasleitung 40 ein Kalibriergasstrom 42 in das Messgerät 20 eingeleitet. Dieser Kalibriergasstrom 42 wird alternierend entweder dem ersten Sensor 22 oder dem zweiten Sensor 24 ohne weitere Aufbereitung, lediglich gedrosselt, zugeführt.

Die aus den Sensoren 22, 24 austretenden Gasströme werden über eine Vorrichtung 44 zusammengeführt und der Volumenstrom in Summe gemessen. Durch einzelnes schalten der Ventile kann somit der Durchfluss in jedem einzelnen Zweig gemessen werden.

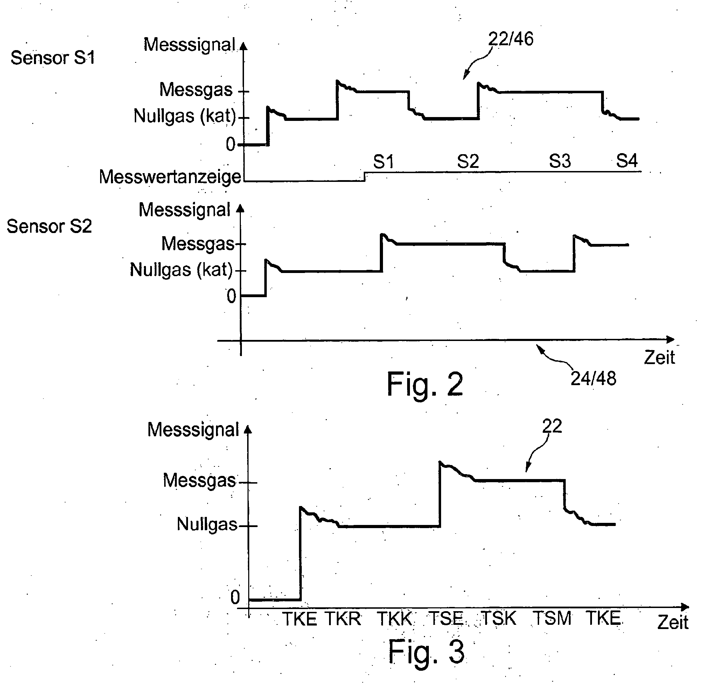

Figur 2 verdeutlicht in zwei Gaswegschaltschemata eine mögliche Funktionsweise des Messgeräts 20. Dem ersten Sensor 22 ist ein erstes Gaswegschaltschema 46 und dem zweiten Sensor 24 ein zweites Gaswegschaltschema 48 zugeordnet. Dargestellt sind die Gasströme und eine bevorzugte Messwertanzeige über die Zeit.

Zu Anfang, beispielsweise beim Start des Messgeräts 20 werden in einer Anlaufphase zunächst in beiden Gaswegen die Sensoren 22, 24 mit dem jeweiligen Referenzgasstrom 34, 36 beaufschlagt. Nach einiger Zeit schaltet der Gasweg des ersten Sensors 22 um und beaufschlagt den ersten Sensor 22 mit Messgas. Der Sensor 22 führt eine oder mehrere Messungen durch und erzeugt ein Messergebnis, das als Anzeigewert S1 angezeigt wird. Anschließend wird dem ersten Sensor 22 wieder Referenzgas zugeführt. Der gleiche Messzyklus wird zeitversetzt beim zweiten Sensor 24 durchgeführt, der einen Anzeigewert S2 erzeugt.

Erkennbar ist, dass die Anzeigewerte S1, S2 zeitversetzt zum Gaswechsel der Sensoren 22,24 angezeigt werden. Hierdurch wird erreicht, dass das Messgerät 20 bzw. eine entsprechende Anzeigeeinheit kontinuierlich einen Anzeigewert anzeigen kann.

Figur 3 verdeutlicht in einem Messzyklusschema beispielhaft einen Messzyklus des ersten Sensors 22. Zu Anfang wird nach Zufuhr des ersten Referenzgasstroms 34 ein Einschwingen des Signals über der Zeit TKE erzeugt. Es folgt das Spülen mit Referenzgas während eines stabilen Betriebszustands TKR. In der anschließenden Kalibrierphase TKK werden die ermittelten Messergebnisse beispielsweise mit bei der Herstellung für diesen Sensor 22 erfassten Messergebnissen verglichen und gegebenenfalls kalibriert. Denkbar ist auch ein Abgleich des Messergebnisses mit gleichartigen Messergebnissen des zweiten Sensors 24.

Nach einem Umschalten auf Messgas folgt eine erneute Einschwingphase über den Zeitraum TSE. Während des anschließenden stabilen Betriebszustands TSK erfolgt eine Kalibrierung oder der Abgleich des ersten Sensors 22 mit dem unmittelbar zuvor gemessenen Messergebnis des zweiten Sensors 24 (TSM). Die Einschwingphase kann beispielsweise auch ein verzögerter Messwertanstieg sein.

Der Wert TSM des Sensors 22 und Sensors 24 wird von der Anzeigeeinheit dargestellt.

Nach erneutem Umschalten auf den ersten Referenzgasstrom 34 startet der Messzyklus erneut und wiederholt sich fortlaufend.

## Patentansprüche

1. Messgerät (20) zur Erfassung von Kohlenwasserstoffanteilen in Gasen, aufweisend
- Vorrichtungen (28) zum Aufteilen eines zu messenden Ursprungsgasstroms (26) in einen ersten Messgasstrom (38) und einen zweiten Messgasstrom (39),
- eine erste Referenzgaseinheit (30) zur Erzeugung eines ersten Referenzgasstroms (34) aus einem Teilstrom des ersten Messgasstroms (38),
- eine zweite Referenzgaseinheit (32) zur Erzeugung eines zweiten Referenzgasstroms (36) aus einem Teilstrom des zweiten Messgasstroms (39),
- einen ersten Sensor (22) zur Bestimmung des Antells an Kohlenwasserstoff im ersten Messgasstrom (38) und zur Erzeugung eines entsprechenden ersten Messergebnisses,
- einen zweiten Sensor (24) zur Bestimmung des Anteils an Kohlenwasserstoff im zweiten Messgasstrom (39) und zur Erzeugung eines entsprechenden zweiten Messergebnisses,
- eine Auswerteinheit zur Auswertung der Messergebnisse der beiden Sensoren (22, 24),
wobei
- dem ersten Sensor (22) im Wechsel der erste Messgasstrom (38) oder der von der ersten Referenzgaseinheit (30) aufbereitete erste Referenzgasstrom (34) zugeführt wird,
- dem zweiten Sensor (24) im Wechsel der zweite Messgasstrom (39) oder der von der zweiten Referenzgaseinheit (32) aufbereitete zweite Referenzgasstrom (36) zugeführt wird.
- die Zyklen der Sensoren (22, 24) derart zeitversetzt sind, dass ein kontinuierliches Messsignal bereitsteht.

2. Messgerät (20) nach Anspruch 1, **dadurch gekennzeichnet, dass** die Sensoren (22, 24) durch Photoionisationsensoren gebildet sind.

3. Messgerät (20) nach Anspruch 1, **dadurch gekennzeichnet, dass** die Sensoren (22, 24) durch Metalloxidsensoren gebildet sind.

4. Messgerät (20) nach einem der Ansprüche 1 bis 3, **dadurch gekennzeichnet, dass** die Referenzgaseinheiten (30, 32) als Katalysatoren, insbesondere als Oxidationskatalysatoren ausgebildet sind.

5. Messgerät (20) nach einem der Ansprüche 1 bis 4, **dadurch gekennzeichnet, dass** im Verlauf der Gasleitungen (40) Elemente aus der Gruppe Drosseln, Ventile (41) oder Durchflussreduktoren vorgesehen sind.

6. Messgerät (20) nach einem der Ansprüche 1 bis 5, **dadurch gekennzeichnet, dass** über eine weitere Gasleitung (40) ein Kalibriergasstrom (42) in das Messgerät (20) einleitbar ist.

7. Messgerät (20) nach 6, **dadurch gekennzeichnet, dass** der Kalibriergasstrom (42) alternierend entweder dem ersten Sensor (22) oder dem zweiten Sensor (24) zuführbar ist.

8. Messgerät (20) nach einem der Ansprüche 1 bis 7, **dadurch gekennzeichnet, dass** die Referenzgaseinheit (32) integraler Bestandteil des Messgerätes (20) ist.

9. Messgerät (20) nach einem der Ansprüche 1 bis 8, **gekennzeichnet durch** zwei räumlich getrennte Baugruppen, eine Sensoreinheit mit Sensoren (22, 24) und eine Auswerteeinheit mit Bedienoberfläche.

10. Verfahren zum Erfassen des Kohlenwasserstoffantells in einem Gasstrom, **gekennzeichnet durch** die Verfahrensschritte,
- Aufteilen eines zu messenden Ursprungsgasstroms (26) in einen ersten Messgasstrom (38) und einen zweiten Messgasstrom (39),
- Erzeugen eines ersten Referenzgasstroms (34) aus einem Teilstrom des ersten Messgasstroms (38),
- Erzeugen eines zweiten Referenzgasstroms (36) aus einem Teilstrom des zweiten Messgasstroms (39),
- Wechselseitiges Zuführen des ersten Messgasstroms (38) oder des von der ersten Referenzgaseinheit (30) aufbereiteten ersten Referenzgasstroms (34) zum ersten Sensor (22),
- Wechselseitiges Zuführen des zweiten Messgasstrom (39) oder des von der zweiten Referenzgaseinheit (32) aufbereiteten zweiten Referenzgasstroms (36) zum zweiten Sensor (24),
- Bestimmen des Anteils an Kohlenwasserstoff im ersten Messgasstrom (38) und Erzeugen eines entsprechenden ersten Messergebnisses,
- Bestimmen des Anteils an Kohlenwasserstoff im zweiten Messgasstrom (39) und Erzeugen eines entsprechenden zweiten Messergebnisses,
- Auswerten der Messergebnisse der beiden Sensoren (22, 24), wobei
- zu Anfang in einer Anlaufphase zunächst in beiden Gaswegen die Sensoren (22, 24) mit dem jeweiligen Referenzgasstrom (34, 36) beaufschlagt werden,
- nach einiger Zeit der erste Sensors (22) mit Messgas beaufschlagt wird, und der erste Sensor (22) zumindest eine Messung durchführt und ein Messergebnis erzeugt, das als Anzeigewert (S1) angezeigt wird,
- anschließend dem ersten Sensor (22) wieder Referenzgas zugeführt wird, und
- der gleiche Messzyklus, der einen Anzeigewert (S2) erzeugt, zeitversetzt beim zweiten Sensor (24) durchgeführt wird.

11. Verfahren nach Anspruch 10, **dadurch gekennzeichnet, dass** Anzeigewerte (S1, S2) zeitversetzt zum Gaswechsel der Sensoren (22,24) angezeigt werden.

12. Verfahren nach einem der Ansprüche 10 bis 11, **dadurch gekennzeichnet, dass** neben dem unveränderten Messgas und dem Referenzgas weiterhin regelmäßig ein Kalibriergas zugeführt wird.

13. Verfahren nach Anspruch 10 bis 12, **dadurch gekennzeichnet, dass** die Messintervalle in Abhängigkeit von der Gaskonzentration verändert werden, bei geringer Konzentration im ppb-Bereich dauert ein Messintervall eine bis 50 Sekunden, insbesondere ca. 30 Sekunden, bei großen Konzentrationen im ppm-Bereich dagegen etwa 80 bis 150, vorzugsweise etwa 120 Minuten.

14. Verfahren nach Anspruch 10 bis 13, **dadurch gekennzeichnet, dass** das Umschalten der Anzeigewerte zeitversetzt zum Umschalten der Gaswege und zur Beaufschlagung der Sensoren erfolgt.

## Claims

1. A measuring device (20) for detecting hydrocarbon contents in gases, comprising
- devices (28) for dividing an original gas flow (26) to be measured into a first measuring gas flow (38) and a second measuring gas flow (39),
- a first reference gas unit (30) for generating a first reference gas flow (34) from a partial flow of the first measuring gas flow (38),
- a second reference gas unit (32) for generating a second reference gas flow (36) from a partial flow of the second measuring gas flow (39),
- a first sensor (22) for determining the hydrocarbon content in the first measuring gas flow (38) and for producing a corresponding first measurement result,
- a second sensor (24) for determining the hydrocarbon content in the second measuring gas flow (39) and for producing a corresponding second measurement result,
- an evaluation unit for evaluating the measurement results of the two sensors (22, 24),
wherein
- the first measuring gas flow (38) or the first reference gas flow (34) processed by the first reference gas unit (30) are alternately fed to the first sensor (22),
- the second measuring gas flow (39) or the second reference gas flow (36) processed by the second reference gas unit (32) are alternately fed to the second sensor (24),
- the cycles of the sensors (22, 24) are time-shifted in such a manner that a continuous measuring signal is provided.

2. The measuring device (20) according to claim 1, **characterized in that** the sensors (22, 24) are formed by photoionization sensors.

3. The measuring device (20) according to claim 1, **characterized in that** the sensors (22, 24) are formed by metal oxide sensors.

4. The measuring device (20) according to any one of the claims 1 to 3, **characterized in that** the reference gas units (30, 32) are configured as catalysts, in particular as oxidation catalysts.

5. The measuring device (20) according to any one of the claims 1 to 4, **characterized in that** over the course of the gas pipes (40), elements from the group of throttles, valves (41) or flow-through reducers are provided.

6. The measuring device (20) according to any one of the claims 1 to 5, **characterized in that** a calibrating gas flow (42) can be introduced into the measuring device (20) via a further gas pipe (40).

7. The measuring device (20) according to claim 6, **characterized in that** the calibrating gas flow (42) can alternately be fed to either the first sensor (22) or the second sensor (24).

8. The measuring device (20) according to any one of the claims 1 to 7, **characterized in that** the reference gas unit (32) is an integral component part of the measuring device (20).

9. The measuring device (20) according to any one of the claims 1 to 8, **characterized by** two spatially separated assemblies, a sensor unit with sensors (22, 24) and an evaluation unit with an operating interface.

10. A method for detecting the hydrocarbon content in a gas flow,
**characterized by** the method steps:
- dividing an original gas flow (26) to be measured into a first measuring gas flow (38) and a second measuring gas flow (39),
- generating a first reference gas flow (34) from a partial flow of the first measuring gas flow (38),
- generating a second reference gas flow (36) from a partial flow of the second measuring gas flow (39),
- alternately feeding the first measuring gas flow (38) or the first reference gas flow (34) processed by the first reference gas unit (30) to the first sensor (22),
- alternately feeding the second measuring gas flow (39) or the second reference gas flow (36) processed by the second reference gas unit (32) to the second sensor (24),
- determining the hydrocarbon content in the first measuring gas flow (38) and producing a corresponding first measurement result,
- determining the hydrocarbon content in the second measuring gas flow (39) and producing a corresponding second measurement result,
- evaluating the measurement results of the two sensors (22, 24),
wherein
- at the beginning, in a start-up phase, the sensors (22, 24) in both gas paths are first exposed to the respective reference gas flow (34, 36),
- after some time, the first sensor (22) is exposed to measuring gas, and the first sensor (22) carries out at least one measurement and thereby produces a measurement result that is displayed as a read-out value (S1),
- then, reference gas is again fed to the first sensor (22), and
- the same measurement cycle that produces a read-out value (S2) is carried out with a time shift at the second sensor (24).

11. The method according to claim 10, **characterized in that** read-out values (S1, S2) are displayed with a time shift with respect to the gas change of the sensors (22, 24).

12. The method according to any one of the claims 10 to 11, **characterized in that**, in addition to the unchanged measuring gas and the reference gas, a calibration gas is also regularly supplied.

13. The method according to claim 10 to 12, **characterized in that** the measurement intervals are changed dependent on the gas concentration; in the case of a low concentration in the ppb range, a measurement interval lasts one to 50 seconds, in particular approx. 30 second, in the case of large concentrations in the ppm range, however, about 80 to 150, preferably about 120 minutes.

14. The method according to claim 10 to 13, **characterized in that** the read-out values are switched with a time shift with respect to the switching of the gas paths and the exposure of the sensors.

## Revendications

1. Appareil de mesure (20) destiné à détecter des proportions d'hydrocarbures dans des gaz, comprenant
- des dispositifs (28) pour diviser un flux de gaz initial à mesurer (26) en un premier flux de gaz de mesure (38) et un deuxième flux de gaz de mesure (39),
- une première unité de gaz de référence (30) pour générer un premier flux de gaz de référence (34) à partir d'un flux partiel du premier flux de gaz de mesure (38),
- une deuxième unité de gaz de référence (32) pour générer un deuxième flux de gaz de référence (36) à partir d'un flux partiel du deuxième flux de gaz de mesure (39),
- un premier capteur (22) pour déterminer la proportion d'hydrocarbure dans le premier flux de gaz de mesure (38) et pour générer un premier résultat de mesure correspondant,
- un deuxième capteur (24) pour déterminer la proportion d'hydrocarbure dans le deuxième flux de gaz de mesure (39) et pour générer un deuxième résultat de mesure correspondant,
- une unité d'évaluation pour évaluer les résultats de mesure des deux capteurs (22, 24),
dans lequel
- audit premier capteur (22) est amené alternativement le premier flux de gaz de mesure (38) ou le premier flux de gaz de référence (34) traité par la première unité de gaz de référence (30),
- audit deuxième capteur (24) est amené alternativement le deuxième flux de gaz de mesure (39) ou le deuxième flux de gaz de référence (36) traité par la deuxième unité de gaz de référence (32),
- les cycles des capteurs (22, 24) sont décalés dans le temps de telle sorte qu'un signal de mesure continu est à la disposition.

2. Appareil de mesure (20) selon la revendication 1, **caractérisé par le fait que** les capteurs (22, 24) sont formés par des détecteurs à photo-ionisation.

3. Appareil de mesure (20) selon la revendication 1, **caractérisé par le fait que** les capteurs (22, 24) sont formés par des capteurs à oxydes métalliques.

4. Appareil de mesure (20) selon l'une quelconque des revendications 1 à 3, **caractérisé par le fait que** les unités de gaz de référence (30, 32) sont réalisées en tant que catalyseurs, en particulier en tant que catalyseurs d'oxydation.

5. Appareil de mesure (20) selon l'une quelconque des revendications 1 à 4, **caractérisé par le fait que** dans le parcours des conduites de gaz (40) sont prévus des éléments du groupe des papillons, vannes (41) ou réducteurs de débit.

6. Appareil de mesure (20) selon l'une quelconque des revendications 1 à 5, **caractérisé par le fait qu'**un flux de gaz de calibrage (42) peut être introduit dans l'appareil de mesure (20) via une autre conduite de gaz (40).

7. Appareil de mesure (20) selon la revendication 6, **caractérisé par le fait que** le flux de gaz de calibrage (42) peut être amené alternativement soit au premier capteur (22) soit au deuxième capteur (24).

8. Appareil de mesure (20) selon l'une quelconque des revendications 1 à 7, **caractérisé par le fait que** l'unité de gaz de référence (32) fait partie intégrante de l'appareil de mesure (20).

9. Appareil de mesure (20) selon l'une quelconque des revendications 1 à 8, **caractérisé par** deux ensembles séparés dans l'espace, une unité à capteurs ayant des capteurs (22, 24) et une unité d'évaluation à surface de commande.

10. Procédé de détection de la proportion d'hydrocarbures dans un flux de gaz,
**caractérisé par** les étapes de procédé:
- diviser un flux de gaz initial à mesurer (26) en un premier flux de gaz de mesure (38) et un deuxième flux de gaz de mesure (39),
- générer un premier flux de gaz de référence (34) à partir d'un flux partiel du premier flux de gaz de mesure (38),
- générer un deuxième flux de gaz de référence (36) à partir d'un flux partiel du deuxième flux de gaz de mesure (39),
- amener audit premier capteur (22) alternativement le premier flux de gaz de mesure (38) ou le premier flux de gaz de référence (34) traité par la première unité de gaz de référence (30),
- amener audit deuxième capteur (24) alternativement le deuxième flux de gaz de mesure (39) ou le deuxième flux de gaz de référence (36) traité par la deuxième unité de gaz de référence (32),
- déterminer la proportion d'hydrocarbure dans le premier flux de gaz de mesure (38) et générer un premier résultat de mesure correspondant,
- déterminer la proportion d'hydrocarbure dans le deuxième flux de gaz de mesure (39) et générer un deuxième résultat de mesure correspondant,
- évaluer les résultats de mesure des deux capteurs (22, 24),
dans lequel
- au début, dans une phase de démarrage, les capteurs (22, 24) sont alimentés d'abord dans les deux voies de gaz en ledit flux de gaz de référence (34, 36) respectif,
- après un certain temps, le premier capteur (22) est alimenté en gaz de mesure et le premier capteur (22) réalise au moins une mesure et génère un résultat de mesure qui est affiché en tant que valeur d'affichage (S1),
- ensuite, au premier capteur (22) est amené à nouveau du gaz de référence, et
- le même cycle de mesure qui génère une valeur d'affichage (S2) est effectué de manière décalée dans le temps dans le deuxième capteur (24).

11. Procédé selon la revendication 10, **caractérisé par le fait que** des valeurs d'affichage (S1, S2) sont affichées de manière décalée dans le temps par rapport au changement de gaz des capteurs (22, 24).

12. Procédé selon l'une quelconque des revendications 10 à 11, **caractérisé par le fait que**, outre le gaz de mesure inchangé et le gaz de référence, on amène en outre régulièrement un gaz de calibrage.

13. Procédé selon la revendication 10 à 12, **caractérisé par le fait que** les intervalles de mesure sont modifiés en fonction de la concentration de gaz; à une faible concentration dans la plage ppb, un intervalle de mesure dure entre 1 et 50 secondes, en particulier 30 secondes environ, par contre, à des concentrations importantes dans la plage ppm, il dure entre 80 et 150, de préférence 120 minutes à peu près.

14. Procédé selon la revendication 10 à 13, **caractérisé par le fait que** la commutation des valeurs d'affichage se fait de manière décalée dans le temps par rapport à la commutation des voies de gaz et à l'alimentation des capteurs.
